(19) Europäisches Patentamt
European Patent Office
Office européen des brevets

(11) Numéro de publication: **0 097 636**
**B1**

(12) # FASCICULE DE BREVET EUROPEEN

(45) Date de publication du fascicule du brevet: **21.08.85**

(51) Int. Cl.⁴: **C 07 D 471/04**, A 61 K 31/55 // (C07D471/04, 221:00, 209:00)

(21) Numéro de dépôt: **83870061.5**

(22) Date de dépôt: **16.06.83**

(54) **Nouveaux dérivés d'indolizine, leur procédé de préparation ainsi que les compositions thérapeutiques les contenant.**

(30) Priorité: **17.06.82 FR 8210598**

(43) Date de publication de la demande: **04.01.84 Bulletin 84/1**

(45) Mention de la délivrance du brevet: **21.08.85 Bulletin 85/34**

(84) Etats contractants désignés: **AT CH DE FR GB IT LI LU NL SE**

(56) Documents cités: **FR - A - 2 495 616**

(73) Titulaire: **SANOFI, Société dite:, 40, Avenue George V, F-75008 Paris (FR)**

(72) Inventeur: **Rosseels, Gilbert, Rue G.van Campenhout 23, B-1810 Wemmel (BE)**
Inventeur: **Polster, Peter, Rue Champ D'Oiseaux, 9 bis, B-5590 Hamme-Mille (BE)**

(74) Mandataire: **Cauchie, Daniel et al, c/o S.A. LABAZ-SANOFI N.V. Avenue De Béjar, 1, B-1120 Bruxelles (BE)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



BUNDESDRUCKEREI BERLIN

**Description**

La présente invention se rapporte, d'une manière générale, à des dérivés hétérocycliques et plus particulièrement à de nouveaux dérivés d'indolizine ainsi qu'à leur procédé de préparation.

Les dérivés d'indolizine de la présente invention peuvent être représentés par la formule générale:

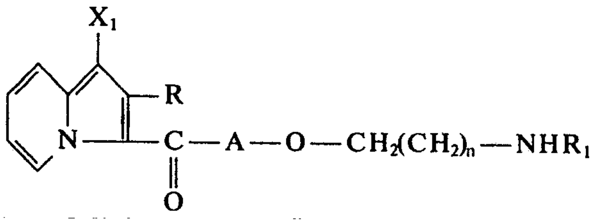

(I)

dans laquelle:

- R représente un radical alkyle linéaire ou ramifié, ayant de 1 à 4 atomes de carbone ou un radical phényle,
- $R_1$ représente un radical alkyle, linéaire ou ramifié, ayant de 1 à 6 atomes de carbone,
- $X_1$ représente hydrogène, chlore, brome, méthyle ou méthoxy,
- A représente un radical de formule:

$R_2$ = 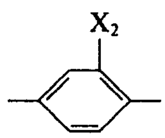 ou $R_3$ = 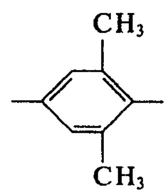

dans laquelle $X_2$ représente hydrogène, chlore, brome, méthyle ou méthoxy,

- n représente 1 ou 2.

L'invention se rapporte également aux sels d'addition non toxiques des composés de formule I, par exemple le chlorhydrate, le bromhydrate ou l'oxalate.

Dans la formule I ci-dessus, R peut représenter un radical méthyle, éthyle, n-propyle, isopropyle, n-butyle, isobutyle ou tertiobutyle, $R_1$ un radical méthyle, éthyle, n-propyle, isopropyle, n-butyle, isobutyle, tertiobutyle, n-pentyle, isopentyle ou néopentyle.

En outre, les composés préférés de formule I sont ceux dans lesquels $R_1$ représente un radical alkyle ramifié par exemple tertiobutyle ou néopentyle. On a trouvé que les dérivés d'indolizine de l'invention possèdent de remarquables propriétés pharmacologiques notamment des propriétés inhibitrices de la translation calcique au niveau membranaire. Ces propriétés sont capables de rendre les composés en question très utiles dans le traitement de certains syndromes pathologiques du coeur, en particulier dans le traitement de l'angine de poitrine, de l'hypertension, de l'arythmie, de l'insuffisance circulatoire cérébrale. Dans le domaine antitumoral, les composés de l'invention pourront être utiles comme potentialisateurs d'anticancéreux.

En conséquence, l'invention se rapporte également à des compositions pharmaceutiques ou vétérinaires contenant, comme principe actif, au moins un dérivé d'indolizine de formule I ou un sel d'addition non toxique de ce dérivé en association avec un véhicule pharmaceutique ou un excipient approprié.

Selon la voie d'administration choisie, la posologie journalière pour un être humain pesant 60 kgs se situera entre 2 et 500 mg de principe actif.

Les composés de formule I peuvent être préparés, selon l'invention, en condensant, dans un milieu inerte tel que par exemple le benzène ou le toluène, une bromoalkoxy-benzoyl indolizine de formule générale:

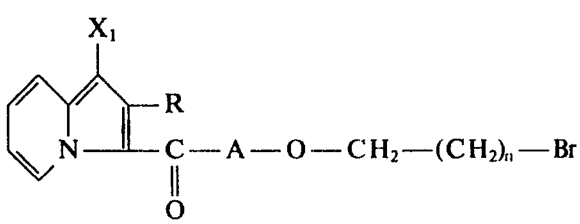

(II)

dans laquelle R, $X_1$, A et n ont la même signification que dans la formule I, avec une amine primaire de formule générale:

$H_2N-R_1$ (III)

dans laquelle $R_1$ a la même signification que dans la formule I, pour former le dérivé souhaité d'indolizine de formule I que l'on fait réagir si on le désire avec un acide organique ou inorganique approprié pour obtenir un sel d'addition non toxique. La condensation en question sera effectuée de préférence à une température comprise entre la température ambiante et 75°C. Les composés de formule II sont des produits connus ayant été décrits dans les brevets français Nos. 2 341 578 et 2 495 616.

L'opinion médicale actuelle estime que la pathophysiologie de l'angor s'étent de la lésion athérosclérotique pure au spasme coronaire pur. On croit cependant que chez de nombreux patients existe une combinaison de la lésion et du spasme.

L'angor dû à une lésion pure peut être prévenu en réduisant la demande en oxygène. L'angor dû au spasme de l'artère coronaire peut être traité en prévenant le spasme lui-même avec pour conséquence une augmentation de l'apport en oxygène.

Suivant cette nouvelle interprétation de l'angine de poitrine, la thérapie optimale antiangineuse devrait fournir cette double action: augmenter l'apport en oxygène tout en réduisant la demande en oxygène.

On sait que les ions calcium agissant au niveau cellulaire contrôlent le degré de vasoconstriction et, par la même occasion, jouent un rôle critique dans la crise d'angine de poitrine.

Les composés antagonistes du calcium agissent au niveau de la membrane cellulaire empêchant sélectivement le calcium d'intervenir dans le processus de contraction au sein de la cellule artérielle.

Grâce à cette action, de tels composés peuvent contrôler:

- l'angor vasospastique en dilatant les artères coronaires pour augmenter l'apport d'oxygène au myocarde et en prévenant le spasme de l'artère coronaire.
- l'angine d'effort en dilatant les artères périphériques pour réduie la tension développée dans la paroi cardiaque et la demande en oxygène du myocarde.
- l'angine de poitrine double qui comporte des caractéristiques de l'angine vasospastique et de l'angine d'effort.

Parmi les produits utilisés dans la prévention chronique de l'angor on peut citer les agents bloquants des récepteurs $\beta$. Les $\beta$-bloquants préviennent d'abord l'angine de poitrine en réduisant la demande en oxygène du myocarde. Tout comme ces agents, les antagonistes du calcium, comme la nifédipine, réduisent la demande en oxygène du muscle cardiaque mais fournissent cependant une dimension accrue au contrôle de l'angor en augmentant l'apport d'oxygène au myocarde lorsque le spasme artériel est présent.

En outre;

- les $\beta$-bloquants peuvent provoquer ce spasme alors que les antagonistes du calcium le préviennent,
- les $\beta$-bloquants diminuent la perfusion cardiaque dans des zones poststénotiques tandis que les antagonistes du calcium augmentent la perfusion cardiaque à la fois aux zones normales et poststénotiques.
- le traitement par les $\beta$-bloquants est limité chez les patients présentant un dysfonctionnement cardiaque, de l'asthme, un blocage du faisceau de His ou du diabète. Les antagonistes du calcium peuvent être administrés en sécurité à tous ces groupes de patients.

D'autre part, certains auteurs s'accordent à penser que l'action cardiodépressive des composés antagonistes du calcium au niveau du myocarde »in situ« est dans une certaine mesure régularisée par un mécanisme de réflexe.

Ce mécanisme de réflexe dont la composante principale emprunte la voie du système $\beta$-adrénergique, aboutit notamment à une augmentation de la puissance et de la vélocité de la contraction cardiaque. Ainsi, chaque fois qu'une chute excessive de la pression artérielle se produit, un réflexe de libération de transmetteurs sympathiques endogènes est déclenché. Si ce système est bloqué, l'insuffisant cardiaque voit s'aggraver sa défaillance cardiaque. Ce relâchement de catécholamines peut neutraliser partiellement l'action cardiodépressive exercée par les substances antagonistes du calcium car contrairement aux inhibiteurs $\beta$-adrénergiques, les agents anticalciques n'inhibent pas la réponse du myocarde aux catécholamines $\beta$-adrénergiques. Par ce mécanisme de compensation adrénergique, les composés antagonistes du calcium pourraient, par conséquent, pallier d'une certaine manière à certains phénomènes indésirables périphériques tels que chute de la pression artérielle contrairement aux inhibiteurs $\beta$-adrénergiques.

On connaît déjà un dérivé d'indolizine à savoir la butoprozine ou éthyl-2 [(di-n-butylamino-3 propyl)oxy-4 benzoyl]-3 indolizine lequel présente à la fois des propriétés antiadrénergiques $\alpha$ et $\beta$ (brevet français No. 2 341 578) c'est-à-dire des propriétés inhibitrices partielles des réactions adrénergiques $\alpha$ et $\beta$ et des propriétés inhibitrices du calcium (Biochemical Pharmacology, vol. 30, No. 8, pp. 897—901, 1981).

Or, on a trouvé, dans le cadre de l'invention, que les composés de formule I ainsi que leurs sels d'addition non toxiques sont doués de propriétés inhibitrices vis-à-vis du calcium tout en étant dépourvus de propriétés antiadrénergiques significatives aux doses où la butoprozine présente déjà de telles propriétés.

Comme l'action inhibitrice sur le système adrénergique des composés de l'invention est très faible ou même nulle, il est permis de supposer, en accord avec le mécanisme décrit précédemment, que l'utilisation de ces composés sera, dans beaucoup de cas, plus avantageuse que celle de la butoprozine.

Il apparaît en conséquence que les composés antagonistes du calcium tels que les composés de l'invention représentent une nouvelle approche du contrôle de l'angor et constituent de ce fait, un apport précieux à la thérapie de l'angine de poitrine notamment dans les cas où il est recommandé de ne pas inhiber les réponses du système adrénergique.

Les résultats de tests pharmacologiques effectués en vue de déterminer les propriétés cardiovasculaires des composés de l'invention sont répertoriés ci-dessous.

## I. Propriétés inhibitrices calciques

Les propriétés inhibitrices de la translocation calcique au niveau membranaire présentées par les composés de l'invention ont été mises en évidence par mesure de leur action antagoniste vis-à-vis de la réponse contractile à la dépolarisation provoquée par le potassium sur l'aorte isolée de rat. Il est bien établi que la dépolarisation de la membrane d'un muscle lisse par le potassium rend cette dernière perméable au calcium extracellulaire et provoque la contraction musculaire. Dès lors, la mesure de l'inhibition de la réponse contractile à la dépolarisation par le potassium ou la mesure d'un relâchement de la contraction tonique à la dépolarisation potassique peut constituer une évaluation de la puissance d'un composé en tant qu'inhibiteur de la perméabilité membranaire aux ions $Ca^{++}$.

Le technique utilisée est la suivante:

Sur des rats mâles Wistar pesant environ 300 g, on prélève l'aorte et on la coupe en bandelettes d'environ 40 mm de longueur et 3 mm de largeur. On place ces fragments dans une cuve à organe isolé de 25 ml contenant une solution de Krebs-bicarbonate modifiée (NaCl 112 m M; KCl 5 m M; $NaHCO_3$ 25 m M; $KH_2PO_4$ 1 m M; $MgSO_4$ 1,2 m M; $CaCl_2$ 2,5 m M; glucose 11,5 m M; eau distillée jusqu'à 1000 ml), parcourue par un courant de carbogène et maintenue à 37° C. On relie la préparation à un microcapteur de force et on enregistre la réponse contractile après amplification sur un enregistreur.

On applique une tension de 2 g à la préparation. On maintient la préparation durant 60 minutes dans la solution de Krebs-bicarbonate modifiée puis on provoque des contractions en remplaçant la solution Krebs-bicarbonate par une solution de Krebs-potassique (NaCl 17 m M; KCl 100 m M; $NaHCO_3$ 25 m M; $KH_2PO_4$ 1 m M; $MgSO_4$ 1,2 m M; $CaCl_2$ 2,5 m M; glucose 11,5 m M; eau distillée jusqu'à 1000 ml). Lorsque la réponse contractile de la préparation est devenue reproductible on introduit, dans le bain, une quantité déterminée d'un composé de l'invention. Soixante minutes plus tard un nouveau spasme est provoqué par la dépolarisation potassique.

Les résultats obtenus sur chaque aorte éprouvée sont alors exprimés en % de l'effet contracturant maximal avant l'incubation avec le composé à tester. A titre d'exemple, les résultats suivants ont été obtenus:

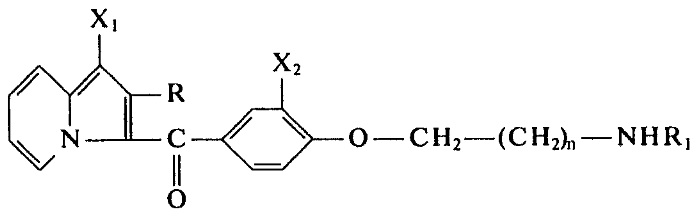

| R | $R_1$ | $X_1$ | $X_2$ | n | Effet contracturant maximal (%) |
|---|---|---|---|---|---|
| a) A la dose de $10^{-6}$ M | | | | | |
| $CH_3$ | $C-(CH_3)_3$ | H | H | 2 | 59 |
| $C_2H_5$ | $C_2H_5$ | H | H | 2 | 66,2 |
| $C_2H_5$ | $C-(CH_3)_3$ | H | H | 2 | 4,4 |
| $C_2H_5$ | $CH_2-C-(CH_3)_3$ | H | H | 2 | 8,6 |
| n-$C_3H_7$ | $C-(CH_3)_3$ | H | H | 2 | 0 |
| n-$C_3H_7$ | $CH_2-C-(CH_3)_3$ | H | H | 2 | 6,2 |
| iso-$C_3H_7$ | n-$C_4H_9$ | H | H | 2 | 25 |
| iso-$C_3H_7$ | $C-(CH_3)_3$ | H | H | 2 | 4,2 |
| iso-$C_3H_7$ | $C-(CH_3)_3$ | Br | H | 2 | 28,6 |
| iso-$C_3H_7$ | $C-(CH_3)_3$ | H | Br | 2 | 11,4 |
| n-$C_4H_9$ | $C-(CH_3)_3$ | H | H | 1 | 65,3 |
| n-$C_4H_9$ | $CH_2-C-(CH_3)_3$ | H | H | 1 | 40,9 |
| n-$C_4H_9$ | $CH_3$ | H | H | 2 | 69,8 |
| n-$C_4H_9$ | $C_2H_5$ | H | H | 2 | 51,6 |
| n-$C_4H_9$ | n-$C_3H_7$ | H | H | 2 | 29,1 |
| n-$C_4H_9$ | iso-$C_3H_7$ | H | H | 2 | 29,4 |
| n-$C_4H_9$ | n-$C_4H_9$ | H | H | 2 | 33 |
| n-$C_4H_9$ | $C-(CH_3)_3$ | H | H | 2 | 7,6 |
| n-$C_4H_9$ | $CH_2-C-(CH_3)_3$ | H | H | 2 | 16,4 |
| n-$C_4H_9$ | n-$C_4H_9$ | Cl | H | 2 | 54,5 |
| n-$C_4H_9$ | $-C-(CH_3)_3$ | Cl | H | 2 | 44,7 |
| n-$C_4H_9$ | $C-(CH_3)_3$ | Br | H | 1 | 61,4 |
| n-$C_4H_9$ | $CH_2-C-(CH_3)_3$ | Br | H | 1 | 66,2 |
| n-$C_4H_9$ | $C-(CH_3)_3$ | Br | H | 2 | 45 |
| n-$C_4H_9$ | $CH_2-C-(CH_3)_3$ | Br | H | 2 | 59,8 |
| n-$C_4H_9$ | $C-(CH_3)_3$ | H | $OCH_3$ | 2 | 3,6 |

Fortsetzung

| R | $R_1$ | $X_1$ | $X_2$ | n | Effet contracturant maximal (%) |
|---|---|---|---|---|---|
| a) A la dose de $10^{-6}$ M | | | | | |
| 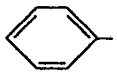 | n-$C_4H_9$ | H | H | 2 | 57,4 |
| 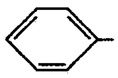 | $C-(CH_3)_3$ | H | H | 2 | 29,8 |
| Butoprozine | | | | | 6,1 |
| b) A la dose de $10^{-7}$ M | | | | | |
| $C_2H_5$ | $C-(CH_3)_3$ | H | H | 2 | 71 |
| $C_2H_5$ | $CH_2-C-(CH_3)_3$ | H | H | 2 | 61,6 |
| iso-$C_3H_7$ | $C-(CH_3)_3$ | H | H | 1 | 89 |
| iso-$C_3H_7$ | $CH_2-C-(CH_3)_3$ | H | H | 1 | 68 |
| iso-$C_3H_7$ | n-$C_4H_9$ | H | H | 2 | 84,5 |
| iso-$C_3H_7$ | $C-(CH_3)_3$ | H | H | 2 | 43,3 |
| iso-$C_3H_7$ | $CH_2-C-(CH_3)_3$ | H | H | 2 | 39 |
| n-$C_4H_9$ | $CH_2-C-(CH_3)_3$ | H | H | 1 | 87 |
| n-$C_4H_9$ | n-$C_3H_7$ | H | H | 2 | 83,4 |
| n-$C_4H_9$ | iso-$C_3H_7$ | H | H | 2 | 78 |
| n-$C_4H_9$ | n-$C_4H_9$ | H | H | 2 | 76,4 |
| n-$C_4H_9$ | $C-(CH_3)_3$ | H | H | 2 | 70 |
| n-$C_4H_9$ | $CH_2-C-(CH_3)_3$ | H | H | 2 | 71,4 |
| n-$C_4H_9$ | $C-(CH_3)_3$ | H | $OCH_3$ | 2 | 85,2 |
| 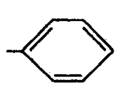 | $C-(CH_3)_3$ | H | H | 2 | 80,4 |
| Butoprozine | | | | | 70,4 |

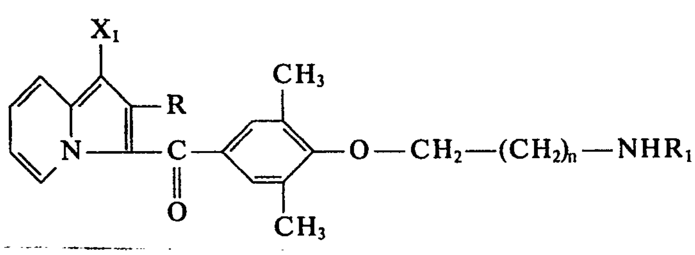

| R | $R_1$ | $X_1$ | n | Dose | Effet contractu-rant maximal (%) |
|---|---|---|---|---|---|
| iso-$C_3H_7$ | iso-$C_3H_7$ | H | 2 | $10^{-6}$ M | 26,5 |
| | | | | $10^{-7}$ M | 68,7 |

II. Propriétés hémodynamiques

Chez le chien, les composés de l'invention provoquent, à la dose de 5 à 10 mg/kg par voie intraveineuse, une diminution de 15 à 40% de la fréquence cardiaque ainsi qu'une chute lente et progressive de la pression artérielle.

En outre des tests effectués également chez le chien à la dose de 5 mg/kg par voie intraveineuse, selon la méthode décrite dans le brevet français No. 2 341 578, ont révélé que les composés de l'invention présentent dans l'ensemble des propriétés antiadrénergiques $\alpha$ de faible intensité, c'est-à-dire généralement inférieure à 50% et des propriétés antiadrénergiques $\alpha$ de faible intensité, c'est-à-dire généralement inférieures à 50% et des propriétés antiadrénergiques $\beta$ d'intensité nulle ou pratiquement nulle.

Cependant, la butoprozine, dans les mêmes conditions montre déjà des propriétés antiadrénergiques $\alpha$ de loin supérieures à 50% et des propriétés antiadrénergiques $\beta$ de l'ordre de 50%.

Les composés de l'invention présentent donc, par rapport à la butoprozine, un spectre d'activités antiadrénergiques nul ou beaucoup moins étendu tout en montrant des propriétés anticalciques du même ordre de grandeur que celles de la butoprozine.

Les compositions thérapeutiques selon l'invention peuvent être présentées sous toute forme convenant à l'administration en thérapie humaine ou vétérinaire. Pour ce qui concerne l'unité d'administration, celle-ci peut prendre la forme, par exemple, d'un comprimé, d'une dragée, d'une capsule, d'une gélule, d'une poudre, d'une suspension ou d'un sirop pour l'administration orale, d'un suppositoire pour l'administration rectale ou d'une solution ou suspension pour l'administration parentérale.

Les compositions thérapeutiques de l'invention pourront comprendre, par unité d'administration, par exemple de 15% à 50% en poids d'ingrédient actif pour l'administration orale, de 3% à 15% d'ingrédient actif pour l'administration rectale et de 3% à 5% d'ingrédient actif pour l'administration parentérale.

Les compositions thérapeutiques de l'invention pourront comprendre, par unité d'administration, par exemple de 15% à 50% en poids d'ingrédient actif pour l'administration orale, de 3% à 15% d'ingrédient actif pour l'administration rectale et de 3% à 5% d'ingrédient actif pour l'administration parentérale.

Suivant la voie d'administration choisie, les compositions pharmaceutiques ou vétérinaires de l'invention seront préparées en associant au moins un des composés de formule I ou un sel d'addition non toxique de ce composé avec un excipient approprié, ce dernier pouvant être constitué par exemple d'au moins un ingrédient sélectionné parmi les substances suivantes: lactose, amidons, talc, stéarate de magnésium, polyvinylpyrrolidone, acide alginique, silice colloïdale, eau distillée, alcool benzylique ou agents édulcorants.

Les Exemples, non limitatifs suivants, illustrent l'invention:

### Exemple 1

Préparation de l'oxalate acide d'éthyl-2 [(tertiobutylamino-3 propyl)oxy-4 benzoyl]-3 indolizine

Dans un ballon, on chauffe pendant 50 heures, à la température de 50° C, une solution constituée de 4,2 g (0,11 mole) d'éthyl-2[(bromo-3 propyl)oxy-4 benzoyl]-3 indolizine et 2,4 g (0,033 mole) de tertiobutylamine dans 40 ml de toluène. Après réaction, on laisse refroidir, verse le mélange réactionnel dans 40 ml d'eau et alcalinise avec une solution aqueuse d'hydroxyde de sodium à 10%. On sépare la phase organique, extrait la solution aqueuse avec du toluène, réunit les phases organiques et lave à l'eau jusqu'à neutralité. On évapore à sec et purifie le résidu huileux par chromatographie d'élution sur oxyde d'aluminium en utilisant du dichloro-1,2 éthane comme éluant.

On dissout le produit huileux purifié dans l'éther éthylique auquel on ajoute une solution éthérée d'acide oxalique anhydre et on recristallise le solide obtenu dans du méthanol.

De cette manière, on obtient 3,9 g d'oxalate acide d'éthyl-2[(tertiobutylamino-3 propyl)oxy-4 benzoyl]-3 indolizine, ce qui représente un rendement de 75%.

P. F.: 207—208° C.

De la même manière que celle décrite ci-dessus on a préparé les composés suivants:

Oxalate acide d'éthyl-2[(éthylamino-3 propyl)oxy-4 benzoyl]-3 indolizine
P. F.: 184° C (éthanol)
Oxalate acide d'éthyl-2[(n-butylamino-3 propyl)oxy-4 benzoyl]-3 indolizine
P. F.: 155° C (acétate d'éthyle/méthanol)
Oxalate acide de n-butyl-2[(tertiobutylamino-3 propyl)oxy-4 benzoyl]-3 indolizine
P. F. 110° C et 143—145° C (méthanol/éther éthylique)
Oxalate acide de n-butyl-2[(n-butylamino-3 propyl)oxy-4 benzoyl]-3 indolizine
P. F.: 167—168° C (méthanol/éther éthylique)
Oxalate acide d'éthyl-2[(n-butylamino-3 propyl)oxy-4 benzoyl]-3 indolizine
P. F.: 172—173° C (isopropanol)
Oxalate acide d'isopropyl-2[(n-butylamino-3 propyl)oxy-4 benzoyl]-3 indolizine
P. F.: 168—170° C (éthanol)
Oxalate acide d'isopropyl-2[(tertiobutylamino-3 propyl)oxy-4 benzyl]-3 indolizine
P. F.: 195—197° C (acétate d'éthyle/méthanol)
Chlorhydrate d'isopropyl-2[(tertiobutylamino-3 propyl)-oxy-4 benzoyl]-3 indolizine
P. F.: 238° C (acétate d'éthyle/méthanol)
Oxalate acide de phényl-2[(n-butylamino-3 propyl)oxy-4 benzoyl]-3 indolizine
P. F.: 210—211° C (diméthylformamide)
Oxalate acide de phényl-2[(tertiobutylamino-3 propyl)oxy-4 benzoyl]-3 indolizine
P. F.: 205° C (méthanol/éther éthylique)
Oxalate acide de n-butyl-2[(méthylamino-3 propyl)oxy-4 benzoyl]-3 indolizine
P. F.: 132° C (méthyl éthyl cétone/méthanol)
Oxalate acide de n-butyl-2[(tertiobutylamino-2 éthyl)oxy-4 benzoyl]-3 indolizine
P. F.: 205—208° C (méthanol/éther éthylique)
Oxalate acide d'éthyl-2[(néopentylamino-3 propyl)oxy-4 benzoyl]-3 indolizine
P. F.: 210° C (diméthylformamide)
Oxalate acide de n-butyl-2[(n-propylamino-3 propyl)oxy-4 benzoyl]-3 indolizine
P. F.: 155—157° C (isopropanol)
Oxalate acide de n-butyl-2[(éthylamino-3 propyl)oxy-4 benzoyl]-3 indolizine
P. F.: 171—172° C (méthanol)
Oxalate acide de n-butyl-2[(isopropylamino-3 propyl)oxy-4 benzoyl]-3 indolizine
P. F.: 159—160° C (isopropanol)
Oxalate acide de n-butyl-2[(néopentylamino-3 propyl)oxy-4 benzoyl]-3 indolizin
P. F.: 190—191° C (méthanol)
Oxalate acide de n-butyl-2[(néopentylamino-2 éthyl)oxy-4 benzoyl]-3 indolizine
P. F.: 197—198° C (méthanol)
Bromhydrate d'isopropyl-2[(tertiobutylamino-2 éthyl)oxy-4 benzoyl]-3 indolizine
P. F.: 222° C (éthanol/éther éthylique 2/1)
Oxalate acide d'isopropyl-2[(néopentylamino-2 éthyl)oxy-4 benzoyl]-3 indolizine
P. F.: 205—207° C (méthanol)
Oxalate acide d'isopropyl-2[(néopentylamino-3 propyl)oxy-4 benzoyl]-3 indolizine
P. F.: 206—207° C (méthanol)
Oxalate acide de méthyl-2[(tertiobutylamino-3 propyl)oxy-4 benzoyl]-3 indolizine
P. F.: 215° C (méthanol)
Oxalate acide de n-propyl-2[(néopentylamino-3 propyl)oxy-4 benzoyl]-3 indolizine
P. F.: 190—192° C (méthanol)
Oxalate acide de chloro-1 n-butyl-2[(tertiobutylamino-3 propyl)oxy-4 benzoyl]-3 indolizine
P. F.: 168° C (éthanol)
Oxalate acide de chloro-1 n-butyl-2[(n-butylamino-3 propyl)oxy-4 benzoyl]-3 indolizine
P. F.: 164° C (éthanol)
Oxalate acide de n-propyl-2[(tertiobutylamino-3 propyl)oxy-4 benzoyl]-3 indolizine
P. F.: 182—184° C (méthanol)
Oxalate acide de bromo-1 n-butyl-2[(néopentylamino-2 éthyl)oxy-4 benzoyl]-3 indolizine
P. F.: 195—197° C (méthanol)
Oxalate acide de bromo-1 n-butyl-2[(tertiobutylamino-2 éthyl)oxy-4 benzoyl]-3 indolizine
P. F.: 220—222° C (méthanol)
Oxalate acide de bromo-1 n-butyl-2[(tertiobutylamino-3 propyl)oxy-4 benzoyl]-3 indolizine
P. F.: 168—170° C (méthanol)
Bromhydrate de bromo-1 n-butyl-2[(néopentylamino-3 propyl)oxy-4 benzoyl]-3 indolizine
P. F.: 184—186° C (méthyl éthyl cétone/méthanol)
Oxalate acide de bromo-1 phényl-2[(n-butylamino-3 propyl)oxy-4 chloro-3 benzoyl]-3 indolizine
P. F.: 176—178° C (méthanol)

Chlorhydrate de bromo-1 isopropyl-2[(tertiobutylamino-3 propyl)oxy-4 benzoyl]-3 indolizine
P. F.: 207—209° C (éther éthylique/méthanol 9/1)
Oxalate acide de n-butyl-2[(tertiobutylamino-3 propyl)oxy-4 méthoxy-3 benzoyl]-3 indolizine
P. F.: 168° C (éthanol)
Oxalate acide d'isopropyl-2[(tertiobutylamino-3 propyl)oxy-4 diméthyl-3,5 benzoyl]-3 indolizine
P. F.: 244° C (méthanol)
Chlorhydrate d'isopropyl-2[(tertiobutylamino-3 propyl)oxy-4 bromo-3 benzoyl]-3 indolizine
P. F.: 238° C (méthanol/éther éthylique)

Exemple 2

Suivant des techniques pharmaceutiques connues, on a préparé une capsule contenant les ingrédients suivants:

| Ingrédient | mg |
|---|---|
| Composé de l'invention | 100,0 |
| Amidons | 99,5 |
| Silice colloïdale | 0,5 |
| | 200,0 |

**Revendications**

1. Dérivés d'indolizine correspondant à la formule générale:

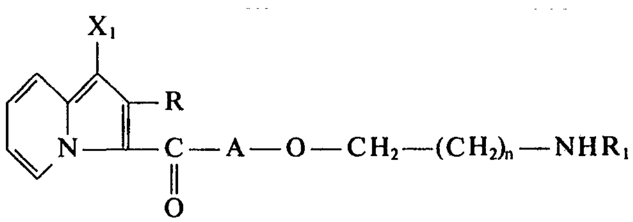

ainsi que leurs sels d'addition non toxiques dans laquelle:

R représente un radical alkyle linéaire ou ramifié, ayant de 1 à 4 atomes de carbone ou un radical phényle,
$R_1$ représente un radical alkyle, linéaire ou ramifié, ayant de 1 à 6 atomes de carbone,
$X_1$ représente hydrogène, chlore, brome, méthyle ou méthoxy,
A représente un radical de formule:

$R_2$ = 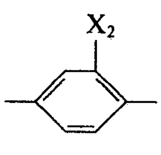 ou $R_3$ = 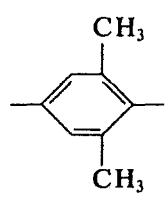

dans laquelle $X_2$ représente hydrogène, chlore, brome, méthyle ou méthoxy,
n représente 1 ou 2.

2. Dérivés d'indolizine selon la Revendication 1 caractérisés en ce que le sel d'addition non toxique est le chlorhydrate, le bromhydrate ou l'oxalate acide.

3. Isopropyl-2[(tertiobutylamino-3 propyl)oxy-4 benzoyl]-3 indolizine et ses sels d'addition non toxiques.

4. n-Propyl-2[(tertiobutylamino-3 propyl)oxy-4 benzoyl]-3 indolizine et ses sels d'addition non toxiques.

5. n-Propyl-2[(néopentylamino-3 propyl)oxy-4 benzoyl]-3 indolizine et ses sels d'addition non toxiques.

6. Isopropyl-2[(néopentylamino-3 propyl)oxy-4 benzoyl]-3 indolizine et ses sels d'addition non toxiques.

7. Isopropyl-2[(tertiobutylamino-3 propyl)oxy-4 bromo-3 benzoyl]-3 indolizine et ses sels d'addition non toxiques.

8. Dérivés d'indolizine selon l'une quelconque des revendications 3 à 7 caractérisés en ce que le sel d'addition non toxique est le chlorhydrate, le bromhydrate ou l'oxalate acide.

9. Chlorhydrate d'isopropyl-2[(tertiobutylamino-3 propyl)oxy-4 benzoyl]-3 indolizine.

10. Procédé de préparation de dérivés d'indolizine selon la Revendication1 caractérisé en ce que l'on condense dans un milieu inerte et à une température comprise entre la température ambiante et 75° C, une bromoalkoxybenzoyl indolizine de formule générale:

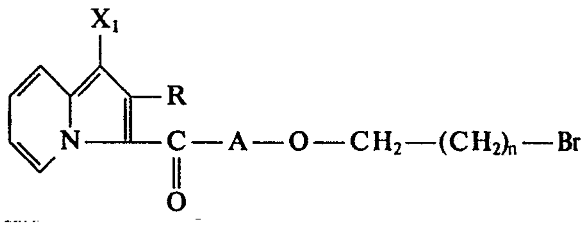

dans laquelle R, $X_1$, A et n ont la même signification que dans la Revendication 1, avec une amine primaire de formule générale:

$H_2N-R_1$

dans laquelle $R_1$ a la même signification que dans la Revendication 1 pour former le dérivé souhaité d'indolizine que l'on fait réagir, si on le désire, avec un acide organique ou inorganique approprié pour obtenir un sel d'addition non toxique.

11. Composition pharmaceutique ou vétérinaire caractérisée en ce qu'elle comprend, comme principe actif, au moins un dérivé d'indolizine selon la Revendication 1 ou 2, en association avec un véhicule pharmaceutique ou un excipient approprié.

12. Composition pharmaceutique ou vétérinaire caractérisée en ce qu'elle comprend, comme principe actif, au moins un dérivé d'indolizine selon l'une quelconque des Revendications 3 à 9, en association avec un véhicule pharmaceutique ou un excipient approprié.

**Patentansprüche**

1. Indolizinderivate gemäß der allgemeinen Formel

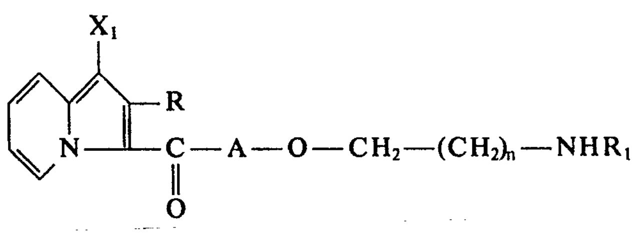

und die pharmazeutisch annehmbaren Säureadditionssalze derselben, worin

R für einen geraden oder verzweigtkettigen Alkylrest mit 1 bis 4 Kohlenstoffatomen oder einen Phenylrest steht,
$R_1$ für einen geraden oder verzweigtkettigen Alkylrest mit 1 bis 6 Kohlenstoffatomen steht,
$X_1$ Wasser, Chlor, Brom, Methyl oder Methoxy bedeutet,
A für einen Rest der Formel

$R_2 =$ 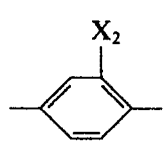 oder $R_3 =$ 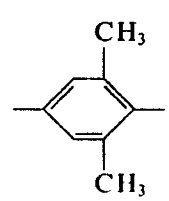

steht, in welcher $X_2$ Wasserstoff, Chlor, Brom, Methyl oder Methoxy bedeutet und n 1 oder 2 bedeutet.

2. Indolizinderivate nach Anspruch 1, in welchen die pharmazeutisch annehmbaren Säureadditionssalze das Hydrochlorid, das Hydrobromid oder das saure Oxalat sind.

3. 2-Isopropyl-3-[4-(3-tert.-butylaminopropyl)-oxy-benzoyl]-indolizin und die pharmazeutisch annehmbaren Säureadditionssalze desselben.

4. 2-n-Propyl-3-[4-(3-tert.-butylaminopropyl)-oxy-benzoyl]-indolizin und die pharmazeutisch annehmbaren Säureadditionssalze desselben.

5. 2-n-Propyl-3-[4-(3-neopentylaminopropyl)-oxy-benzoyl]-indolizin und die pharmazeutisch annehmbaren Säureadditionssalze desselben.

6. 2-Isopropyl-3-[4-(3-neopentylaminopropyl)-oxy-benzoyl]-indolizin und die pharmazeutisch annehmbaren Säureadditionssalze desselben.

7. 2-Isopropyl-3-[4-(3-tert.-butylaminopropyl)-oxy-3-brom-benzoyl]-indolizin und die pharmazeutisch annehmbaren Säureadditionssalze desselben.

8. Indolizinderivate nach einem der Ansprüche 3 bis 7, in welchen das pharmazeutisch annehmbare Säureadditionssalz das Hydrochlorid, das Hydrobromid oder das saure Oxalat ist.

9. 2-Isopropyl-3-[4-tert.-butylaminopropyl)-oxy-benzoyl]-indolizin-hydrochlorid.

10. Verfahren zur Herstellung eines Indolizinderivates nach Anspruch 1, dadurch gekennzeichnet, daß man einem inerten Medium und bei einer Temperatur zwischen Raumtemperatur und 75°C ein Bromalkoxy-benzoyl-indolizin der allgemeinen Formel

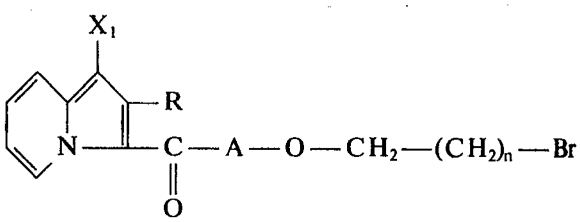

worin R, $X_1$, A und n dieselbe Bedeutung wie in Anspruch 1 haben, mit einem primären Amin der allgemeinen Formel

$$H_2N-R_1$$

in welcher $R_1$ dieselbe Bedeutung wie in Anspruch 1 hat, zur Bildung des erforderlichen Indolizinderivates kondensiert, das gegebenenfalls mit einer geeigneten organischen oder anorganischen Säure zur Bildung eines pharmazeutisch annehmbaren Säureadditionssalzes desselben umgesetzt wird.

11. Pharmazeutische oder veterinärmedizinische Zusammensetzung, enthaltend als wesentlichen aktiven Bestandteil mindestens ein Indolizinderivat nach Anspruch 1 oder 2 in Verbindung mit einem pharmazeutischen Träger oder Streckmittel dafür.

12. Pharmazeutische oder veterinärmedizinische Zusammensetzung, enthaltend als wesentlichen aktiven Bestandteil mindestens ein Indolizinderivat nach Anspruch 3 bis 9 in Verbindung mit einem pharmazeutischen Träger oder Streckmittel dafür.

**Claims**

1. Indolizine derivatives corresponding to the general formula:

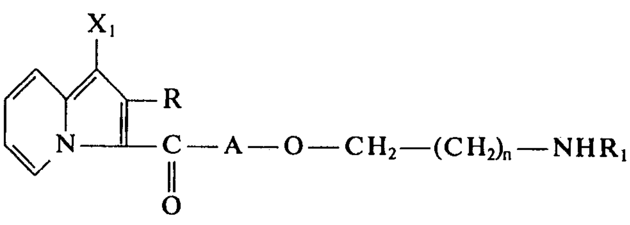

and the pharmaceutically acceptable acid addition salts thereof, wherein:

R represents a straight- or branched-chain alkyl radical having from 1 to 4 carbon atoms or a phenyl radical,
$R_1$ represents a straight- or branched-chain alkyl radical having from 1 to 6 carbon atoms,
$X_1$ represents hydrogen, chlorine, bromine, methyl or methoxy,
A represents a radical of the formula:

$R_2$ = 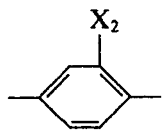 or $R_3$ = 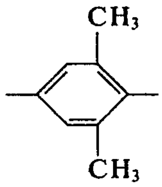

in which $X_2$ represents hydrogen, chlorine, bromine, methyl or methoxy n represents 1 or 2.

2. Indolizine derivatives as claimed in Claim 1 wherein the pharmaceutically acceptable acid addition salts is the hydrochloride, the hydrobromide or the acid oxalate.

3. 2-Isopropyl-3-[4-(3-tert-butylamino-propyl)-oxy-benzoyl]-indolizine and pharmaceutically acceptable acid addition salts thereof.

4. 2-n-Propyl-3-[4-(3-tert-butylamino-propyl)-oxy-benzoyl]-indolizine and pharmaceutically acceptable acid addition salts thereof.

5. 2-n-Propyl-3-[4-(3-neopentylamino-propyl)-oxy-benzoyl]-indolizine and pharmaceutically acceptable acid addition salts thereof.

6. 2-Isopropyl-3-[4-(3-neopentylamino-propyl)-oxy-benzoyl]-indolizine and pharmaceutically acceptable acid addition salts thereof.

7. 2-Isopropyl-3-[4-(3-tert-butylamino-propyl)-oxy-3-bromo-benzoyl]-indolizine and pharmaceutically acceptable acid addition salts thereof.

8. Indolizine derivatives as claimed in any one of Claims 3 to 7 wherein the pharmaceutically acceptable acid addition salt is the hyrochloride, the hydrobromide or the acid oxalate.

9. 2-Isopropyl-3-[4-(3-tert-butylamino-propyl)-oxy-benzoyl]-indolizine hydrochloride.

10. A process for preparing an indolizine derivative as claimed in Claim 1 which process comprises condensing, in an inert medium and at a temperature between room-temperature and 75° C, a bromalkoxy-benzoyl-indolizine of the general formula:

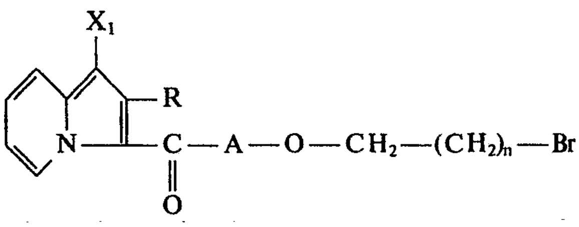

wherein R, $X_1$, A and n have the same meaning as in Claim 1 with a primary amine of general formula:

$H_2N-R_1$

in which $R_1$ has the same meaning as in Claim 1 to form the required indolizine derivative which, if desired, is reacted with an appropriate organic or inorganic acid to provide a pharmaceutically acceptable acid addition salt thereof.

11. A pharmaceutical or veterinary composition containing, as essential active ingredient, at least one indolizine derivative as claimed in Claim 1 or 2 in association with a pharmaceutical carrier or excipient therefor.

12. A pharmaceutical or veterinary composition containing, as essential active ingredient, at least one indolizine derivative as claimed in Claims 3 to 9 in association with a pharmaceutical carrier or excipient therefor.